# EUROPEAN PATENT SPECIFICATION

(11) **EP 1 143 981 B1**
(45) Date of publication and mention of the grant of the patent: **09.08.2006**
(21) Application number: 99967738.8
(22) Date of filing: 29.12.1999
(51) Int. Cl.: A61K 31/70, A61P 31/18

(54) **USE OF N-GLYCOLNEURAMINIC ACID AND ITS DERIVATIVES FOR THE MANUFACTURE OF A MEDICAMENT FOR THE TREATMENT OF HIV INFECTIONS**
VERWENDUNG VON N-GLYCOLNEURAMINSÄURE UND DEREN DERIVATEN ZUR HERSTELLUNG EINES MEDIKAMENTS ZUR BEHANDLUNG VON HIV-INFEKTIONEN
UTILISATION DE L'ACIDE N-GLYCOLNEURAMINIQUE ET LEUR DERIVES POUR LA PREPARATION D'UN MEDICAMENT POUR LE TRAITMENT DES INFECTIONS A VIH

(30) Priority: 29.12.1998 US 114540 P
(43) Date of publication of application: 17.10.2001
(73) Proprietor: Glukor, Inc., McLean, VA 22102 (US)
(72) Inventor: SHARMA, Yash, Vienna, VA 22182 (US)
(74) Representative: Jones, Helen M.M.
(86) International application number: PCT/US1999/031161
(87) International publication number: WO 2000/038697

(56) References cited:
- US-A- 4 774 327
- US-A- 5 834 423
- LOBERT ET AL.: 'Cell membrane bound N-acetylneuraminic acid is involved in the infection of fibroblasts and phorbol-ester differentiated monocyte-like cells with human cytomegalovirus (HCMV)' ARCH. VIROL., vol. 140, 1995, pages 1357 - 1371, XP002925281
- SCHULTZE ET AL.: 'Transmissible gastroenteritis coronavirus, but not the related porcine respiratory coronavirus, hasasialic acid (N-glycolylneuraminic acid) binding activity' JOURNAL OF VIROLOGY, vol. 70, no. 8, August 1996, pages 5634 - 5637, XP002925282
- SUZUKI ET AL.: 'Swine influenza virus strains recognize sialylsugar chains containing the molecular species of sialic acid predominantly present in the swine tracheal epithelium' FEBS LETTERS, vol. 404, 1997, pages 192 - 196, XP002925283
- WERNER ET AL.: 'Appropiate Mammalian Expression Systems for Biopharmaceuticals' ARZNEIM-FORSCH., vol. 48, no. 8, August 1998, pages 870 - 880, XP002925284

## Description

### CROSS REFERENCE TO RELATED APPLICATIONS

This application is a continuation-in-part of U.S. Serial No. 09/015,830, filed January 29, 1998 and claims priority from U.S. Provisional Application Serial No. 60/114,540, filed December 29, 1998.

### BACKGROUND OF THE INVENTION

Promising treatments for human immunodeficiency virus-type (HIV) have been developed over the past few years, including combination therapy with protease inhibitors. The cost associated with such treatments is prohibitive, however, as the spread of Acquired Immune Deficiency Syndrome (AIDS) is concentrated in regions of the world with limited financial resources. Although the AIDS incidence and mortality have been decreasing in the United States, it is estimated that 16,000 people worldwide are being infected with HIV each day. In certain African countries, infection rates have reached 25%. See, Balter, M., Science, 1998, 280:1863-1864. Treatment success also has been limited by poor tolerance of the treatments by patients and the emergence of resistant strains of HIV. Thus, a need exists for an effective HIV treatment that is well tolerated and relatively inexpensive.

### SUMMARY OF THE INVENTION

The invention is based on the discovery that N-glycolylneuraminic acid and the sulphated or phosphonylated derivative thereof can be used to prevent or treat viral infections, as well as other pathogenic infections. N-glycolylneuraminic acid is a complex galactose molecule that is produced in many non-human mammals. N-glycolylneuraminic acid was identified from extracts of baboon peripheral blood monocytes (PBMCs) that were capable of inhibiting HIV-1 replication in and/or infection of human cells. As N-glycolylneuraminic acid is a carbohydrate, toxicity is minimal. Thus, the invention provides a safe and effective treatment of HIV.

In one aspect, the invention features N-glycolylneuraminic acid for medicine, e.g., N-glycolylneuraminic acid for treating HIV infections and use of N-glycolylneuraminic acid in the manufacture of a medicament for the treatment of HIV infections.

The invention also features a method for preventing or treating a HIV infection in a subject. The method includes administering N-glycolylneuraminic acid or a derivative thereof, e.g., phosphorylated N-glycolylneuraminic acid or sulfated N-glycolylneuraminic acid, to the subject in an amount effective to prevent or treat the HIV infection. N-glycolylneuraminic acid can be synthetic or extracted from a biological sample. N-glycolylneuraminic acid or the derivatives according to claim 1 can be administered intravenously, subcutaneously, orally, by inhalation, or transdermally. The method further can include monitoring the subject for the presence of the viral infection.

The amount of N-glycolylneuraminic acid or the derivatives according to claim 1 can be about 1 mg to about 1000 mg per administration, about 10 mg to about 100 mg per administration, or about 30 mg to about 80 mg per administration. N-glycolylneuraminic acid or the derivative thereof can be administered daily.

In yet another aspect, the invention features a method for treating a HIV infection in a subject that includes administering a first anti-viral agent and a second anti-viral agent to the subject in amounts effective to treat the HIV infection, wherein the first anti-viral agent is N-glycolylneurammic acid or a derivative according to claim 1. The second anti-viral agent can be, for example a reverse transcriptase inhibitor or a protease inhibitor. The first and second anti-viral agents can be conjugated to each other.

Other features and advantages of the invention will be apparent from the following detailed description, and from the claims.

### BRIEF DESCRIPTION OF THE DRAWINGS

Figure 1 is a graph that illustrates reverse transcriptase assay for HIV infected CEM-TART cells treated with N-glycolylneuraminic acid.
Figure 2 is a chromatogram that depicts the HPLC Profile of LUKOR (C18 column; 0.1% TFA/water and a 0-100% ACN gradient; UV detection A280).
Figures 3A-3C are graphs that illustrate the reverse transcriptase activity in infected cells treated with LUKOR, N-glycolylneuraminic acid, and N-acetylneuraminic acid, respectively.

### DETAILED DESCRIPTION

The invention provides a use according to present claim 1 N-glycolylneuraminic acid (C₁₁H₁₉NO₁₀, MW 325.3) is the hydroxylated derivative of N-acetylneuraminic acid (sialic acid), which is the most prevalent sialic acid in humans. N-glycolylneuraminic acid typically is not detectable in humans. CMP-N-acetylneuraminic acid hydroxylase, the enzyme that converts N-acetylneuraminic acid to N-glycolylneuraminic acid, is not detectable in humans as the gene encoding this enzyme is inactivated by a 92 base pair deletion that results in a frameshift mutation. See, Irie and Suzuki, Biochem. Biophys. Res. Commun., 1998, 248(2):330-333; and Chou et al., Proc. Natl. Acad. Sci. USA, 1998, 95(20):11751-11756. Without being limited to a particular mechanism, the presence of N-glycolylneuraminic acid on the cell surface in non-human primates may provide immunity to HIV infection in such species.

### Preparation of N-glycolylneuraminic acid or derivatives thereof

As used herein, "derivative" refers to a compound that is similar in structure to N-glycolylneuraminic acid. Additional derivatives include phosphorylated or sulfated N-glycolylneuraminic acid, N-glycolylneuraminic acid salts.

N-glycolylneuraminic acid can be purchased commercially from, for example, Sigma Chemical Company, St. Louis, MO. N-glycolylneuraminic acid also can be synthesized. For example, CMP-N-acetylneuraminic acid hydroxylase can be used to synthesize N-glycolylneuraminic acid as its CMP-glycoside. See, Schlenzka et al., Glycobiology, 1994, 4(5):675-683. Non-enzymatic methods of synthesis include, for example, synthesis from N-acetylneuraminic acid using methanol or hydrochloric acid and benzylalcohol. Other synthesis methods are described in Choi et al., J. Org. Chem., 1996, 61:8/39 (from mannosamine), Faillard et al., J. Physiol. Chem., 1965, 344:167 (from glucosamine), U.S. Patent No. 4,774,326, and U.S. Patent No.4,774,327.

Alternatively, N-glycolylneuraminic acid can be purified from biological samples from non-human mammals (e.g., pigs or baboons). In particular, N-glycolylneuraminic acid can be purified from pig submaxillary glands or baboon PBMCs. Generally, PBMCs are isolated from whole blood using, for example, Ficol-Hypaque density-gradient centrifugation. Cells are lysed in a hypotonic solution, e.g., sterile, distilled water. Proteins and nucleic acids are removed from the lysed cells by precipitation, for example, with a stabilizing solution containing 10% v/v calcium chloride and potassium chloride in phosphate-buffered saline, pH 7.4. After removal of the precipitate by centrifugation, the supernatant can be clarified by filtration through a 0.22µm filter and sterilized. This purification procedure removes substantially all macromolecule components from the extract, leaving N-glycolylneuraminic acid and other small molecular weight components. Extracts at this stage of purification have potent antiviral activity.

Further purification procedures such as high-pressure liquid chromatography (HPLC) can be used isolate N-glycolylneuraminic acid from the extract. For example, HPLC can be performed using a C18 column with a mobile phase of 0.1% tetrafluoroacetic acid (TFA) in water and a gradient of 0-100% acetonitrile (ACN).

### Use

Use according to the invention includes administering N-glycolylneuraminic acid or a derivative thereof to a subject in an amount effective to prevent or treat the HIV infection. N-glycolylneuraminic acid or a derivative thereof can be administered to either subjects at risk for a HIV infection or subjects that have been infected. Subjects that are at risk for infection include, for example, health care workers that may have been exposed to a HIV virus through a needle stick or other patient contact, or patients involved in high-risk activities such as intravenous drug use. N-glycolylneuraminic acid or derivatives thereof are useful for treatment or prevention of HIV.

The concentration of N-glycolylneuraminic acid or a derivative thereof effective for preventing or treating a HIV infection in a subject may vary, depending on a number of factors, including the preferred dosage of the compound to be administered, the chemical characteristics of the compounds employed, the formulation of the compound excipients and the route of administration. The optimal dosage of N-glycolylneuraminic acid to be administered also may depend on such variables as the overall health status of the particular patient and the relative biological efficacy of the compound selected.

Typically, about 1 mg to about 1000 mg of N-glycolylneuraminic acid or a derivative thereof is administered to the subject. For example, about 10 mg to about 100 mg or about 30 mg to about 80 mg of N-glycolylneuraminic acid or a derivative thereof can be administered. N-glycolylneuraminic acid or a derivative thereof can be administered as a single, daily dose or administered multiple times over the course of a day. In addition, delayed release formulations can be used such that administrations of N-glycolylneuraminic acid or a derivative thereof are less frequent. N-glycolylneuraminic acid or a derivative thereof can be administered by any route of administration including, for example, orally, intravenously, subcutaneously, transdermally, or by inhalation.

One advantage of using carbohydrate-based therapy is that carbohydrates do not naturally kill the pathogens, but simply prevent them from binding to a cell. Therefore, the presently described therapy should not engender the type of resistance that pathogens typically develop against therapeutics. This profound advantage allows carbohydrate treatment to be used not only in the treatment, but also in the prevention of infection.

The method of the invention further can include monitoring the subject for the presence of the HIV infection. HIV Viral load of the subject (i.e., amount of virus/ ml of blood) can be determined using polymerase chain reaction (PCR) assays (e.g., Roche Amplicor HIV-1 or branched DNA assays (e.g., Chiron Quantiplex) to detect copies of viral RNA. P24 antigen (for HIV) also can be assessed as a measure of viral load. Monitoring of treatment also can include determining the subject's number of CD4 cells, CD4 percentage, number of CD8 cells, CD8 percentage, or ratio of CD4 cells/CD8 cells.

In some embodiments, the use according to claim 1 further includes administering N-glycolylneuraminic acid in combination with other compounds such as polypeptides or therapeutic agents. Non-limiting examples of therapeutic agents include antibiotics, chemotherapy agents, and other antiviral agents such as reverse transcriptase inhibitors (e.g., AZT, ddI, ddC, d4T, and 3TC) or protease inhibitors (e.g., NEVIROAPINE, SAQUINAVIR, RITNOVIR, and INDINARVIR). N-glycolylneuraminic acid also can be conjugated to other compounds or therapeutic agents using standard methodologies and administered to a subject.

### Pharmaceutical Compositions

N-glycolylneuraminic acid or a derivative thereof may be formulated into pharmaceutical compositions by admixture with pharmaceutically acceptable excipients or carriers. Such compounds and compositions may be prepared for parenteral administration, particularly in the form of liquid solutions or suspensions in aqueous physiological buffer solutions; for oral administration, particularly in the form of tablets or capsules; or for intranasal administration, particularly in the form of powders, nasal drops, or aerosols. Compositions for other routes of administration may be prepared as desired using standard methods.

Formulations for parenteral administration may contain as common excipients sterile water or saline, polyalkylene glycols such as polyethylene glycol, oils of vegetable origin, hydrogenated naphthalenes, and the like. In particular, biocompatible, biodegradable lactide polymer, lactide/glycolide copolymer, or polyoxethylene-polyoxypropylene copolymers are examples of excipients for controlling the release of a compound of the invention *in vivo.* Other suitable parenteral delivery systems include ethylene-vinyl acetate copolymer particles, osmotic pumps, implantable infusion systems, and liposomes. Formulations for inhalation administration may contain excipients such as lactose, if desired. Inhalation formulations may be aqueous solutions containing, for example, polyoxyethylene-9-lauryl ether, glycocholate, and deoxycholate, or they may be oily solutions for administration in the form of nasal drops. If desired, the compounds can be formulated as gels to be applied intranasally. Formulations for parenteral administration also may include glycocholate for buccal administration.

### EXAMPLES

**Example 1 ― Isolation of a Small Molecule from Baboon Blood:** Peripheral blood monocytes (PBMC) were isolated from whole baboon blood using Ficoll-Hypaque density gradient centrifugation or from PBMCs further expanded in tissue culture following activation with phytohemagglutinin -P (PHA-P) and growth in medium containing interleukin-2 (IL-2). In either case, the PBMCs first were washed 3 times with sterile phosphate-buffered saline (PBS) and pelleted by centrifugation. The cell pellet then was lysed by resuspension in sterile H₂O and held for 96 hours at 4°C. Proteins and nucleic acids were precipitated from the extract and the remaining components in the extract were stabilized using 10% (v/v) calcium phosphate buffer (pH 7.4) containing 0.01 % calcium chloride and 0.001% ascorbic acid. The solution was clarified by centrifugation followed by filtration through a 0.22µm filter. This final filtrate represented a 1:50 dilution of the initial cell lysate and is hereafter referred to as LUKOR. In some instances, the LUKOR preparation was sterilized by Cobalt radiation at 2.5 mRADs for 3 hours (Neutron Products, Inc. Gaithersburg, MD).

The biological activity of LUKOR was evaluated by determining if p24 expression from HIV-infected human mononuclear cells was inhibited. Human PBMCs were obtained from the American Red Cross and incubated with ~100 TCID₅₀ (tissue culture infective dose) of HIV-1 (Strain IIIB) at 37°C for 3 hours. Excess free virus was removed at the end of the incubation period by washing the cells two times with PBS. Uninfected control PBMCs were cultured in RPMI (Difco) plus 10% bovine calf serum, 100 U/ml penicillin, and 100 µg/ml streptomycin, at 37°C and 5% CO₂. A standard preparation of LUKOR was serially diluted 1:2, 1:4, 1:8, 1:16, 1:32, and 1:64 in saline, and 750µl of each dilution were added to each well containing 750 µL medium and 2 x 20⁵ infected cells. Positive controls (HIV-infected PBMCs with 750 µL of medium in the absence of LUKOR), and negative controls (non-infected PBMCs in the presence of LUKOR) were included in each experiment. An HIV-1 p24 Antigen Kit (Dupont, Wilmington, DE) was used to determine the p24 output in all cell samples after 5 days in culture by measuring optical density of the cultures at 490 nm. Typical results with LUKOR isolated directly from whole blood PBMCs or from PBMCs expanded in tissue culture are shown in Table 1.

**TABLE 1**

| **p24 Expression in Infected Human PBMCs in the Presence or Absence of LUKOR** | | |
|---|---|---|
| Sample | LUKOR Dilution (v/v) | p24 Expression (pg HIV p24/ml) |
| Negative Control | + LUKOR | 97 |
| (uninfected cells) | | |
| Positive Control (infected cells) | - LUKOR | 10,850,000 |
| WB*-LUKOR | 1:2 | 320 |
| WB- LUKOR | 1:8 | 871 |
| WB- LUKOR | 1:16 | 824 |
| WB- LUKOR | 1:64 | 865 |
| TC**-LUKOR | 1:100 | 480 |
| TC-LUKOR | 1:200 | 660 |
| TC-LUKOR | 1:400 | 380 |
| TC-LUKOR | 1:800 | 620 |

| | | |
|---|---|---|
| *LUKOR prepared from PBMCs of whole baboon blood (WB-Lukor) | | |
| **LUKOR prepared from PBMCs expanded in tissue culture (TC-Lukor) | | |

The data in Table 1 indicate that at all dilutions used (up to 1:64 for the whole baboon blood and 1:800 dilution for tissue culture LUKOR), LUKOR was effective in reducing expression of p24. This suggests that LUKOR can inhibit HIV replication. Furthermore, LUKOR was active both from baboon PBMCs isolated from fresh blood, as well as from baboon PBMCs maintained in tissue culture for 4 weeks. Similar results also were seen with lysates stored for 6 weeks at 4°C, suggesting that the active component or components were fairly stable to refrigerated storage conditions. Activity of the extracts also was maintained after acid treatment or boiling of the extract (Table 2), as measured by reverse transcriptase activity. Results in Table 2 are presented as "% inhibition". Similar extracts made from rabbit and human PBMCs did not significantly inhibit production of p24 at dilutions ranging from 1:4 to 1:250 (see Table 2).

**TABLE 2**

| **Comparison of Extracts from Baboon, Humans, and Rabbits** | | | | | | |
|---|---|---|---|---|---|---|
| **Lysate** | **Day** | **Dilution** | | | | **Control** |
| | | **1:4** | **1:20** | **1:100** | **1:250** | |
| **Baboon PBMC** | 5 | 8.6 | 63.6 | 49.2 | 45.7 | 115 |
| **PHA-Baboon PBMC** | 5 | 17 | 26 | 48 | 41 | |
| **Human PBMC** | 5 | 4.7 | 3.4 | 5.7 | 4.7 | |
| **Rabbit PBMC** | 5 | 1.3 | 2.5 | 1.7 | 1.2 | |
| **Buffer-no cells** | 5 | 1.4 | 1.3 | 1.8 | 1.8 | |
| **Baboon PBMC (boiled)** | 5 | 9 | 29 | 72 | 41 | |
| **Baboon PMBC (acid treated)** | 5 | 3 | 31 | 42 | 99 | |

The transmission of HIV-1 from infected T-cells to uninfected T-cells during cocultivation in the presence of LUKOR also was evaluated by p24 production. Supernatants from SUPT-1 T-cells infected with HIV-1 strains GT63 or GT65 were added to uninfected SUPT-1 cells, which were isolated from human blood. After 48 hours, the cells were treated with LUKOR for 1 hour, washed with fresh medium, and cultured for 5 days at which time virus replication was measured by assaying HIV-1 p24 in the supernatants. The infectivities of both strains of HIV-1 (GT63 and GT65) were significantly depressed by treatment of the infected cells for one hour with either a 1:10 or 1:5 dilution with LUKOR (Table 3).

**TABLE 3**

| **Inhibition of Cellular Infectivity by LUKOR** | | |
|---|---|---|
| Treatment | HIV-1 (GT63)* | HIV-1 (GT65)* |
| 1:5 (v/v) LUKOR | 25,600 | 837 |
| 1:10 (v/v) LUKOR | 353,000 | 1,770 |
| No LUKOR | 9,680,000 | 4,478,000 |

| | | |
|---|---|---|
| *Results are expressed in picograms of HIV-1 p24/ml | | |

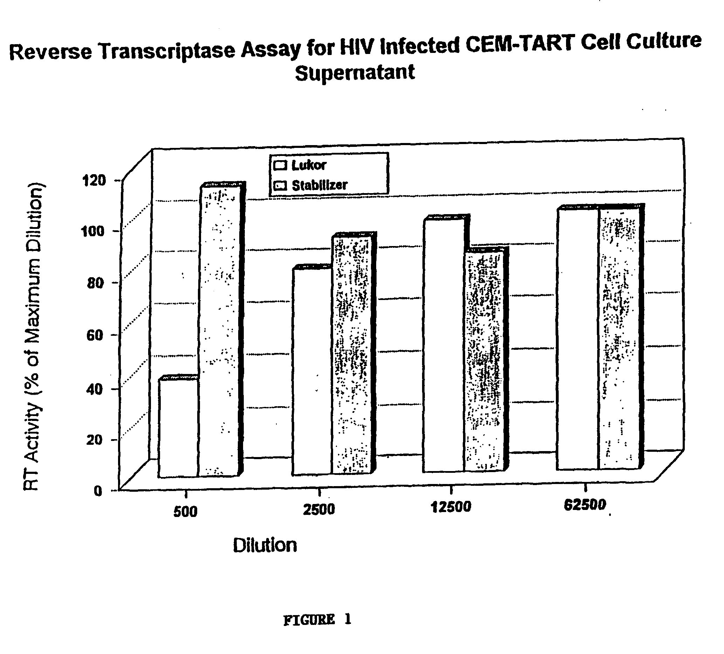

Reverse transcriptase activity was measured after detergent treatment of cell supernatants, by determining [³H]-TTP incorporation into poly(rA):oligo(dT)(rAdT) or by [³H]-GTP incorporation into poly(rC):oligo(dG)((rCdG) homopolymer template/primer systems. See, Walker et al., J. Virol., 1991, 65(11):5921-5927. The reverse transcriptase inhibitors AZTTP (3'-Azido-2',3'-dideoxythymidine-5'-triphosphate) and UC38 (NSC 629243, non-nucleoside reverse transcriptase inhibitor) served as positive controls. Rice, W. G., et.al. 1993, Proc Natl Acad Sci USA 90:9721-9724. Cells (CEM-TART, NIH AIDS reagent program, catalog #136) were treated overnight with various amounts of LUKOR prior to incubation for 6 days with a clone of HIV-1. A dilution of 1:500 (LUKOR from PBMCs in cell culture) consistently resulted in 60-70% inhibition of HIV RT activity (Figure 1). This compared favorably to 1.6nM concentrations of AZT. Dilutions of LUKOR greater than 1:500 (>1:1,000) did not exhibit significant RT inhibitory activity.

HIV-protease activity also was assayed in the presence or absence of various dilutions of LUKOR. The assay contained HIV-1 protease (1.25µg/ml), 0.5 M KHPO₄ buffer (pH 6.5), 5% glycerol, 3mM DTT, 0.5mM EDTA, and 0.75M ammonium sulfate, and was initiated by the addition of the sp211 substrate (Ala-Ser-Gln-Asn-Tyr-Pro-Ile-Val-Gln-amide) to attain a final concentration of 0.1044 mg/ml. The reaction proceeded at 37°C for 30 minutes and then was quenched with 80mM Guanidine-HCl in TFA. The amount of sp211 substrate remaining at the termination of the reaction was determined by reverse phase HPLC. The effect of LUKOR on the inhibition of HIV protease is shown in Table 4.

**TABLE 4**

| **Inhibition of HIV protease by LUKOR from Baboon PBMCs From Cell Culture** | |
|---|---|
| **Dilution of LUKOR** | **Inhibition of HIV Protease (%)** |
| Undiluted | 33% |
| 1:10 | 26% |
| 1:100 | 42% |
| 1:1000 | 13% |
| No LUKOR | 0% |

The specificity of the anti-viral activity of LUKOR was tested against various strains of HIV and SIV-1 (simian immunodeficiency virus). The target cells were human PBMCs that had been incubated with a specified virus overnight. Following incubation, the cells were washed and plated in the presence of various dilutions of LUKOR. One-half of the medium in each well was changed at 3-4 day intervals. Antigen capture was determined with the HIV p24 OTC kit (Organon) at day 10. The control included HIV-infected PBMCs in the absence of LUKOR. Results of these experiments are presented in Table 5 as ng/ml p24, with % inhibition in parentheses. In spite of low levels of p24 expression in HIV-1 strains NS1 (non-syncytium inducing) and SI (syncytium inducing), dilutions of LUKOR caused significant inhibition of viral p24 expression in HIV-1 NSI and SI, H9/IIIB, and SIV-1 (MAC 251).

**TABLE 5**

| **Inhibition of p24 Production in Infected PBMCs** | | | | |
|---|---|---|---|---|
| **Virus** | **Dilution** | | | |
| | **1:4** | **1:20** | **1:100** | **Control** |
| HIV-1 (NSI) | 5.3 (-60) | 3.7 (-12) | 0.96 (71) | 3.3 |
| | 1.9 (-58) | 1.4 (-16) | 0.468 (61) | 1.2 |
| HIV-1 (SI) | 3.9 (-2) | 2.9 (40) | 3.6 (6) | 3.8 |
| H9/IIIB | 427 (4) | 457 (-3) | 385 (13) | 442 |
| SIV-1(MAC251) | 6.5 (97) | 85 (69) | 54 (80) | 271 |

A series *of in vitro* and *in vivo* toxicology studies were conducted to evaluate the potential adverse effects of LUKOR on human blood cells, blood clotting factors, and in rats administered intravenous LUKOR repeatedly over 28 days. Freshly drawn heparinized whole blood (1ml) was diluted 1:4 in PBS (pH 7.4) and 0.1 ml of LUKOR (freshly prepared) was added per ml of diluted whole blood. Two aliquots of the blood were maintained at 4°C for 21 and 42 days. Extract was not added to the control samples (freshly drawn blood, and blood stored for 21 and 42 days). Results are reported in Table 6. LUKOR had no effect on red blood cell (RBC) count, white blood cell (WBC) count, hemoglobin (Hb), hematocrit (Hct), mean corpuscular volume (MCV), mean cell hemoglobin (MCH), RBC osmotic fragility, and hemolysis. The control blood sample that was stored for 42 days began to deteriorate. Reference values were 5.4 million/mm³ RBCs, 12.7g/100ml for Hg content, and 33.3% for Hct (volume of cells in 100ml of blood).

**TABLE 6**

| **Addition of LUKOR to whole blood** | | | | | | |
|---|---|---|---|---|---|---|
| **Parameter** | **Day O** | | **Day 21** | | **Day 42** | |
| | **Untreated** | **Treated** | **Untreated** | **Treated** | **Untreated** | **Treated** |
| **RBC** | 5.3 | 5.3 | 4.2 | 5.1 | 3.0 | 5.1 |
| **Hb** | 12.8 | 12.9 | 8.4 | 12.8 | 6.3 | 12.6 |
| **Hct** | 32.3% | 32.3% | 20.2% | 33.0% | 15.8% | 32.0% |
| **Lysis** | 0% | 0% | 10% | 0% | 40% | 0% |
| **45% saline** | 0% | 0% | 92% | 0% | 58.4% | 0% |

The effects of LUKOR on Factor VIII and Factor IX clotting activities were determined by adding LUKOR (1:10 dilution) to human plasma and incubating the samples for 1, 2, 5, and 23 hours at 37°C. The samples were frozen and assayed for Factor VIII and Factor IX clotting activity by a one-stage APTT method using 0.15 M sodium chloride as the control. Denson, Br. J. Haematol., 1973, 24(4):451-461. Samples also were incubated at room temperature and at 4°C. Factor VIII and Factor IX activities of the samples treated with LUKOR (0.076 and 0.389 units/ml respectively) were not different from saline controls (Table 7, % activity remaining).

**TABLE 7**

| **Clotting Activity in Treated Samples** | | | | |
|---|---|---|---|---|
| **Time (Hrs)** | **FVIII/saline** | **FVIII/LUKOR** | **FIX/Saline** | **FIX/LUKOR** |
| 0 | 91.81 | 111.11 | 91.13 | 93.01 |
| 1 | 93.92 | 114.29 | 98.77 | 103.06 |
| 2 | 80.11 | 120.91 | 96.95 | 93.83 |
| 5 | 93.5 | 114.29 | 93.51 | 93.68 |
| 23 | 67.95 | 87.45 | 40.14 | 62.03 |

*In vivo* toxicology of LUKOR was examined using Sprague-Dawley CD albino rats. Five male rats and 5 female rats were administered 1 ml of LUKOR per day by intravenous injection into the tail vein on 6 occasions (on Days 1, 3, 5, 7, 9 and 28). All animals were examined twice daily for mortality and signs of ill health or reaction to treatment, with a more detailed examination performed weekly. There were no treatment-related clinical signs and the animals did not develop hypersensitivity reactions by the end of the experiment on Day 28. No treatment-related effects on food consumption or weight gain were observed. Blood samples were collected under anesthesia from the orbital sinus, 24 hours following the dose at day 9, and again from the abdominal aorta at necropsy at day 28. All animals were euthanized by exsanguinations from the abdominal aorta following anesthesia. At the termination of the study on Day 28, hematological evaluations were performed on all animals. There were no mortalities, no treatment-related clinical observations, or effects on white cell parameters. Mean values for female animals generally were lower than male animal, but the difference was insignificant considering the normal range. Therefore, it appears that LUKOR can be administered intravenously.

The above studies indicate that an aqueous soluble material can be extracted from baboon PBMCs. This material is active in reducing HIV-infection or replication *in vitro.* Without being limited to a particular mechanism, N-glycolylneuraminic acid may not directly inhibit HIV-1 protease or reverse transcriptase.

### Example 2 - Cytotoxicity of LUKOR on Cultured Blood Mononuclear Cells:

Cultured human blood mononuclear cells were cultured in the presence of varying concentrations of LUKOR for 7 days. Solutions containing different concentrations of LUKOR were prepared by diluting the stock solution of LUKOR (1mg/mL) 1:4, 1:20, 1:100, and 1:500 with PBS. An equal volume of each dilution of LUKOR was added to cultured cells. Medium was changed at Day 3. The resulting cell counts at each concentration of LUKOR are listed in Table 8. A colorimetric assay was used to assess cytotoxicity. A WST-1 test kit (a tetrazolium compound that is the sodium salt of 4-[3-(4-iodophenyl)-2-(4-nitrophenyl)-2H-5-tetrazolio]-1,3-benzene disulfonate) was used in this assay (Roche Diagnostics, Indianapolis, IN). As indicated in Table 8, no cytotoxicity was observed and cell count was maintained at varying concentrations of LUKOR.

**TABLE 8**

| **Cytotoxicity of LUKOR** | | |
|---|---|---|
| Dilution (1X) | WST-1 (%) | Cell Count (%) |
| 0 | 100 | 100 |
| 4 | 84 | 78.8 |
| 20 | 98 | 106 |
| 100 | 101 | 100 |
| 500 | 104 | 106 |

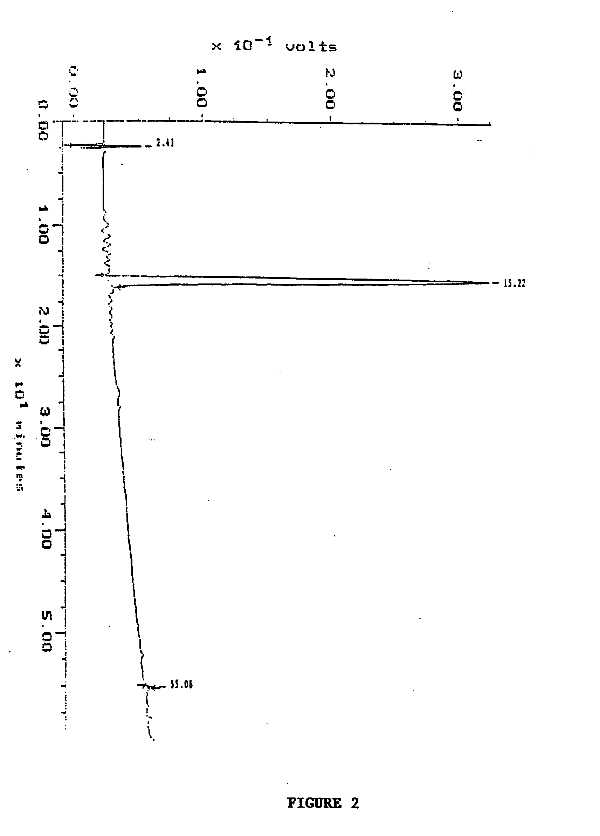

**Example 3 - Identification of Active Component from LUKOR: The** soluble lysate isolated from PBMCs (LUKOR) was fractionated by HPLC as a first step in the identification of the active component. A C18 column (Delta Pak, 15µm, 300Å, 0.39x30 cm) with a mobile phase of 0.1% tetrafluoroacetic acid (TFA) in water and a gradient of 0-100% acetonitrile (ACN) was used to separate the components in the cell lysate. One major peak eluting with 30% ACN and two minor peaks at 50% ACN were observed (Figure 2). The 3 peaks, designated HPLC-1, HPLC-2, and HPLC-3, were collected separately and lyophilized and stored for further characterization by mass spectrometry and NMR.

Mass spectrometry was performed with a VG BioQ triple quadrupole mass spectrometer operating in the positive ion electrospray ionization mode using the following parameters: scan range m/z 100-950 and 35-700; cone voltage 57V to 63V; source temperature 80°C to 100°C. Calibration was performed with direction injection analysis of CsI prior to LC-MS. A distinct aromatic ring absorbance with a peak maximum at 258 nm was detected. Behavior of the compound was consistent with a small molecular weight compound. Sample related masses of 86, 194, and 288 were identified. The sample related masses of 86 and 194 represented less than 1% of total. It was determined that the sample mass of 288 was composed of carbon, hydrogen, oxygen, and a single nitrogen atom.

Proton NMR also was performed on the extract in a solution of D₂O using a modified Nicolet NT 360 MH3 spectrometer operating with a single 0.5µ sec. excitation pulse and a one second re-cycle delay. Signals were detected with shifts between 3.1-3.7ppm.

The active component of LUKOR was identified as N-glycolylneuraminic acid based on the molecular weight and chemical composition. Data supporting this conclusion are described below.

**Example 4-Specificity of Inhibition:** Research grade purified d-Galactose (CAT# g404, lot 117H00431, FW 180.2, Sigma Chemical Co., St. Louis, MO) was dissolved in saline at a concentration of 5mg/ml. The solution was allowed to stand at room temperature for 15 minutes and mixed by vortex until the galactose was completely dissolved. The galactose solution was divided into two equal aliquots. Stabilizing solution (0.1 ml) was added to one aliquot, then vortexed and incubated at room temperature for 30 minutes. After mixing again, the mixture was centrifuged at 1500g for 30 minutes and the supernatant was filtered through a 0.2µm filter and transferred to a sterile 15 ml tube. The solution was sterilized by gamma radiation (2.5 mega RADs) with 15 minutes exposure. The aliquot without the stabilizing solution was labeled "1A" and the aliquot containing the stabilized solution was labeled "1B". Solutions of N-glycolylneuraminic acid (CAT# g2755, lot # 27H0485, FW325.3, Sigma Chemical Co., 5mg/ml) and galactose-α 1,3-galactose (α1, 3 galactosebios, V-Labs, Inc., Covington, LA, 5mg/ml) were prepared as described for galactose, and were labeled "2A" and "2B" and "3A" and "3B", respectively. Solutions 1 and 2 (galactose and N-glycolylneuraminic acid, respectively) were mixed in equal volumes to prepare solution 4. Each of the test solutions were diluted 1:20 1:40,1:80, and 1:160 in saline. Control solutions were water.

One ml of each of the dilutions of the test solution or control was mixed with an equal volume of HIV-1_{III B} (~50 TCID₅₀ /2x10⁵ cells) to infect activated human PBMCs overnight at 37°C (3x10⁵ cells/dilution). HIV-1_{III B} is a laboratory-adapted strain. The cells then were washed and placed in culture media containing the indicated dilution of the test solution in triplicate wells (1x10³ cells per well, ½ medium changes with media containing the indicated dilutions of test solution were made at ~3 day intervals). The conditioned media were tested for HIV-1 P24 output on day 9 using the DuPont P24 assay kit. The control was infected PBMCs in the absence of test solutions. Results are shown in Table 9 and are measured as the Inhibition% of the control. As seen in Table 9, solutions containing N-glycolylneuraminic acid inhibited P24 output, while galactose and N-acetylneuraminic acid did not.

**TABLE 9**

| **N-Glycolylneuraminic Acid Treatment Inhibits p24 Output** | | | | | |
|---|---|---|---|---|---|
| | DILUTION | | | | |
| SAMPLE | 1:10 | 1:20 | 1:40 | 1:80 | 1:160 |
| **1A** | 4 | 5 | 4 | 2 | 2 |
| **1B** | 7 | 5 | 3 | 4 | 2 |
| **2A** | 94 | 78 | 66 | 41 | 19 |
| **2B** | 93 | 81 | 61 | 37 | 16 |
| **3AA** | 4.3 | 3.6 | 2.2 | 1.4 | 0.4 |
| **3B** | 4.6 | 3.6 | 1.8 | 1.1 | 0.5 |
| **4A** | 97 | 88 | 72 | 58 | 46 |
| **4B** | 94 | 82 | 65 | 56 | 45 |

The test was repeated using test solutions stored at 4°C. In this experiment, the dilutions were mixed with HIV-1 IIIB virus at the time of infecting the activated human PBMCs for 3 hours. The cells then were washed and incubated in triplicate wells (1x10³ cells/well) in the presence of the indicated dilution of the test solution. One half media changes included the test solution dilutions. Conditioned media were tested for P24 output by the antigen capture assay on day 6. Results are shown in Table 10 and are presented as the inhibition % of control (no carbohydrate added).

**TABLE 10**

| **Inhibition % of Control** | | | | | |
|---|---|---|---|---|---|
| | Dilution | | | | |
| SAMPLE | 1:10 | 1:20 | 1:40 | 1:80 | 1:160 |
| 2A | 96 | 78 | 62 | 47 | 17 |
| 2B | 88 | 74 | 59 | 44 | 13 |

In addition, a similar experiment was performed, except that test solutions were added post infection, at the time of plating into the triplicate wells. Results are shown in Table 11.

**TABLE 11**

| **Inhibition % of Control** | | | | | |
|---|---|---|---|---|---|
| | Dilution | | | | |
| SAMPLE | 1:10 | 1:20 | 1:40 | 1:80 | 1:160 |
| 2A | 95 | 75 | 66 | 30 | 16 |
| 2B | 82 | 70 | 61 | 26 | 15 |
| 4A | 88 | 71 | 53 | 32 | 14 |
| 4B | 87 | 70 | 45 | 26 | 11 |

This experiment was repeated using further dilutions of the test solution containing N-glycolylneuraminic acid to determine the endpoint of the inhibition. Results are shown in Table 12. The lowest effective concentration for inhibition of HIV p24 output was 3ng/ml.

**TABLE 12**

| **Inhibition % of Control** | | | |
|---|---|---|---|
| | DILUTION | | |
| SAMPLE | 1:20 | 1:100 | 1:1000 |
| **2A** | 88 | 55 | 7 |
| **2B** | 78 | 42 | 1 |

**Example 5-Efficacy of N-Glycolylneuraminic Acid Treatment in CEM-SS and PBMC cells:** N-glycolylneuraminic acid (approximately 90% purity; MW325.3) was purchased from Sigma Chemicals (St. Louis, MO) and dissolved in dH₂O to make an 80mM stock solution. The stock solution was diluted to a starting concentration of 200µM and nine one-half log serial dilutions thereafter. PBMCs were isolated from fresh human blood obtained commercially from the American Red Cross (Baltimore, Maryland). CEM-SS cells were obtained from the NIH AIDS reagent program. The low passage, lymphotropic clinical isolate ROJO was obtained from a pediatric patient attending the AIDS Clinic at the University of Alabama at Birmingham. Phytohemagglutinin (PHA-P) was obtained from Sigma Chemical Co. (St. Louis, MO) and recombinant IL-2 was obtained from Amgen (San Francisco, CA). As a positive control for antiviral assays, AZT was obtained from the NIH AIDS Reagent Program. HIV-1_{RF} also was obtained from the NIH AIDS reagent program. Tritiated thymidine triphosphate was obtained from New England Nuclear (Boston, MA). Each plate in each experiment contained cell control wells (cells only), virus control wells (cells plus virus), drug toxicity control wells (cells plus drug only), drug colorimetric control wells (drug only) as well as experimental wells (drug plus cells plus virus).

Cell Preparation: Prior to infection, CEM-SS cells were grown in complete tissue culture medium (RPMI 1640 medium with 10% heat-inactivated fetal bovine serum, 2mM L-glutamine, 100 U/mL penicillin, 100 µg/mL streptomycin, 10 µg/mL gentamycin) and transferred to T-150 flasks. On the day preceding the assay, the cells were split 1:2 to assure the cells would be in an exponential growth phase at time of infection. On the day of assay, the cells were washed twice with tissue culture medium and resuspended in fresh tissue culture medium. Total cell and viability counting were performed using a hemacytometer and trypan blue dye exclusion. Cell viability was greater than 95% for the cells to be utilized in the assay. The cells were pelleted and resuspended at 2.5 x 10⁴ cells per mL in tissue culture medium. Cells were added to the drug-containing plates in a volume of 50 µL.

Fresh human PBMCs were isolated from voluntary Red Cross donors, seronegative for HIV and HBV. Leukophoresed blood was diluted 1:1 with Dulbecco's PBS, layered over 14 mL of Ficoll-Hypaque density gradient in a 50mL centrifuge and then centrifuged for 30 minutes at 600 x g. Banded PBMCs were gently aspirated from the resulting interface and subsequently washed twice with PBS by low speed centrifugation. After the final wash, cells were enumerated by trypan blue exclusion and resuspended at 1 x 10⁷ cells/mL in RPMI 1640 supplemented with 15% Fetal Bovine Serum (FBS), 2mM L-glutamine, and 4 µg/mL PHA-P. The cells were incubated for 48-72 hours at 37°C, then centrifuged and reset in RPMI 1640 with 15% FBS, 2mM L-glutamine, 100 U/mL penicillin, 100µg/mL streptomycin, 10 µg/mL gentamycin, and 20 U/mL recombinant human IL-2. PBMCs were maintained in this medium at a concentration of 1-2 x 10⁶ cells/mL with bi-weekly medium changes until used in the assay.

For the standard PBMC assay, PHA-P stimulated cells from at least two normal donors were pooled, diluted in fresh medium to a final concentration of 1 x 10⁶ cells/mL, and plated in the interior wells of a 96 well round bottom microplate at 50 µL/well (5 x 10⁴ cells/well). Test drug dilutions were prepared at a 2X concentration in microfuge tubes and 100 µL of each concentration was placed in appropriate wells in a standard format. A predetermined dilution of virus stock was placed in each test well (50 µL, final MOI ≅ 0.1). Wells with cells and virus alone were used for virus control. Separate plates were prepared identically without virus for drug cytotoxicity studies using an XTT assay system. The PBMC cultures were maintained for seven days following infection, at which time cell-free supernatant samples were collected and assayed for reverse transcriptase activity as described below.

Virus Preparation: Pre-tittered aliquots of HIV-1_{ROJO} and HIV-1_{RF} were removed from the freezer (-80°C) and thawed to room temperature in a biological safety cabinet. The virus was resuspended and diluted into tissue culture medium such that the amount of virus added to each well in a volume of 50 µL will be the amount determined to give complete cell killing at 6 days post-infection. TCID₅₀ calculation by endpoint titration in CEM-SS cells indicated that the multiplicity of infection (MOI) of these assays ranged from 0.005-0.01.

Tetrazolium dye staining of screening plates: After 6 days of incubation (37°C with 5% CO₂), the test plates were analyzed by staining with the tetrazolium dye XTT (2,3-b (2-methoxy-4-nitro-5-sulfophenyl)-2H-tetrazolium -5-carboxanilide). XTT-tetrazolium is metabolized by mitochondrial enzymes of metabolically active cells to a soluble formazan product, allowing the rapid quantitative analysis of the inhibition of HIV-induced cell killing by anti-HIV compounds. The XTT solution was prepared daily as a stock of 1 mg/mL in PBS. Phenazine methosulfate (PMS) solution was prepared at 15 mg/mL in PBS and stored in the dark at -20°C. XTT/PMS stock was prepared immediately before use by diluting the PMS 1:100 into PBS and adding 40 µL per ml of XTT solution. Fifty µls of XTT/PMS were added to each well of the plate and the plate was incubated for 4 hours at 37°C. Plates were sealed with adhesive plate sealers and inverted several times to mix the soluble formazan product and the plate was read spectrophotometrically at 450nm with a Molecular Devices Vmax plate reader. Indices such as %CPE Reduction (cytopathic effects), %Cell Viability, IC₂₅, IC₅₀, IC₉₅, TC₂₅, TC₅₀, and TC₉₅ and other indices were calculated. The use of round bottom microtiter plates allowed rapid macroscopic analysis of the activity of a given test compound by the evaluation of pellet size. The results of the macroscopic observations were confirmed and enhanced by further microscopic analysis.

Table 13 provides the toxicity data as percent cell control (%CC) for PBMCs pretreated with N-glycolylneuraminic acid for 2 hours, then infected with HIV_{ROJO}. As indicated in Table 13, no toxicity was observed at concentrations of at least 200mM, i.e., %CC was around 100% at each tested concentration of N-glycolylneuraminic acid. Similar results were observed for cells simultaneously exposed to N-glycolylneuraminic acid and HIV_{ROJO}.

**TABLE 13**

| **Toxicity of N-glycolylneuraminic Acid Treatment** | | | | | | | | | | |
|---|---|---|---|---|---|---|---|---|---|---|
| **Toxicity Values (XTT- O.D. @ 450/650 nm)** | | | | | | | | | | |
| **Conc.(mM)** | **1** | **0.32** | **0.1** | **0.032** | **0.64** | **2** | **6.4** | **20** | **64** | **200** |
| **Sample 1** | 2.157 | 2.213 | 2.282 | 2.317 | 2.250 | 2.255 | 2.325 | 2.256 | 2.014 | 2.100 |
| **Sample 2** | 2.163 | 2.266 | 2.717 | 2.186 | 2.048 | 2.595 | 2.385 | 2.459 | 2.065 | 2.100 |
| **Sample 3** | 1.965 | 2.230 | 2.960 | 2.903 | 2.866 | 2.929 | 2.944 | 2.631 | 1.984 | 2.100 |
| **Mean** | 2.095 | 2.236 | 2.653 | 2.469 | 2.388 | 2.593 | 2.551 | 2.449 | 2.021 | 2.100 |
| **%CC** | **99.8** | **106.5** | **126.3** | **117.6** | **113.7** | **123.5** | **121.5** | **116.6** | **96.2** | **100.0** |

Reverse Transcriptase Activity Assay: A microtiter based reverse transcriptase (RT) reaction was utilized as described by Buckheit et al., (1991), AIDS Research and Human Retroviruses, 7:295-302. Tritiated thymidine triphosphate (³H-TTP) was suspended in distilled H₂O at 5 Ci/mL. Poly rA and oligo dT were prepared as a stock solution which was kept at -20°C. RT reaction buffer was prepared fresh on a daily basis and contained 125 µL 1M EGTA, 125 µL dH₂O, 110 µL 10% SDS, 50 µL 1M Tris (pH 7.4), 50 µL 1M DTT, and 40 µL 1M MgCl₂. These three solutions were mixed together in a ratio of 2 parts ³H-TTP, 1 part poly rA:oligo dT, and 1 part reaction buffer. Ten microliters of this reaction mixture were placed in a round bottom microtiter plate and 15 µL of virus containing supernatant were added and mixed. The plate was incubated at 37°C in a water bath with a solid support to prevent submersion of the plate and incubated for 60 minutes. After the incubation period, the reaction volume was spotted onto pieces of DE81 paper, washed 5 times for 5 minutes each in a 5% sodium phosphate buffer, 2 times for 1 minute each in distilled water, 2 times for 1 minute each in 70% ethanol, and then dried. Opti-Fluor-O (Packard) was added to each sample and incorporated radioactivity was quantified utilizing a Wallac 1450 MicroBeta Plus liquid scintillation counter. Plates were screened with tetrazolium dye staining as described above.

Table 14 provides a summary of the RT activity in PBMCs infected with HIV from the ROJO isolate. As shown in Table 14A, RT activity in cells pretreated with about 0.01 to 1 mM of N-glycolylneuraminic acid for about 2 hours was significantly reduced (3.7 to 46.2 % virus control, VC). RT activity in cells pretreated with concentrations of N-glycolylneuraminic acid less than 0.0032 mM was similar to the virus control. The IC₅₀ was approximately 0.010mM and the TC₅₀ was >1mM, resulting in a therapeutic index (TI) of >100. Table 14B provides the RT activity in cells simultaneously exposed to drug and HIV. The IC₅₀ was approximately 0.058mM and the TC₅₀ was >1mM, with a TI of >17.25.

**TABLE 14**

| **N-Glycolylneuraminic Acid 2-hour Pretreatment of Infected Human PBMCs** | | | | | | | | | | |
|---|---|---|---|---|---|---|---|---|---|---|
| **A. Reverse Transcriptase (RT) Activity (cpm)** | | | | | | | | | | |
| **Conc. (mM)** | **1** | **0.32** | **0.1** | **0.032** | **0.01** | **0.0032** | **0.001** | **0.00032** | **0.0001** | **0** |
| **Sample 1** | 72.0 | 196.0 | 785.0 | 156.0 | 120.0 | 2381.0 | 1969.0 | 2655.0 | 3776.0 | 2060.0 |
| **Sample 2** | 68.0 | 140.0 | 388.0 | 564.0 | 1265.0 | 3368.0 | 1129.0 | 2078.0 | 3131.0 | 2060.0 |
| **Sample 3** | 88.0 | 104.0 | 204.0 | 965.0 | 1469.0 | 1838.0 | 2503.0 | 1313.0 | 1384.0 | 2060.0 |
| **Mean** | 76.0 | 146.7 | 459.0 | 561.7 | 951.3 | 2529.0 | 1867.0 | 2015.3 | 2763.7 | 2060.0 |
| **% VC** | **3.7** | **7.1** | **22.3** | **27.3** | **46.2** | **122.8** | **90.6** | **97.8** | **134.2** | **100.0** |

| **B. RT Activity (cpm)** | | | | | | | | | | |
|---|---|---|---|---|---|---|---|---|---|---|
| **Conc.(mM)** | **1** | **0.32** | **0.1** | **0.032** | **0.01** | **0.0032** | **0.001** | **0.00032** | **0.0001** | **0** |
| **Sample 1** | 108 | 132.0 | 100.0 | 1814.0 | 2284.0 | 3135.0 | 4950.0 | 4589.0 | 2423.0 | 2992.0 |
| **Sample 2** | 368 | 108.0 | 2531.0 | 2094.0 | 2280.0 | 264.0 | 3912.0 | 3119.0 | 2253.0 | 2992.0 |
| **Sample 3** | 136 | 68.0 | 88.0 | 1694.0 | 1926.0 | 5279.0 | 464.0 | 677.0 | 2369.0 | 2992.0 |
| **Mean** | 204 | 102.7 | 906.3 | 1867.3 | 2163.3 | 2892.7 | 3108.7 | 2795.0 | 2348.3 | 2992.0 |
| **%VC** | **6.8** | **3.4** | **30.3** | **62.4** | **72.3** | **96.7** | **103.9** | **93.4** | **78.5** | **100.0** |

In the CEM-SS cell-based assay system, N-glycolylneuraminic acid (3.2µM to 1 mM) did not display anti-HIV activity when tested against the laboratory-adapted HIV-1_{RF} and HIV-1_{IIIB} virus strains. Pretreatment with N-glycolylneuraminic acid for two hours did not reveal latent antiviral properties of the test compound. The TC₅₀ of N-glycolylneuraminic acid was greater than 1mM in the CEM-SS cell based assay using HIV-1_{RF or IIIB}, whereas for AZT, the TC₅₀ was greater than 1µM. The IC₅₀ of AZT was calculated to be 0.005 - 0.007µM for HIV-1_{RF} and 0.011-0.017µM for HIV-1_{IIIB}.

N-glycolylneuraminic acid did have anti-HIV activity in the PBMC cell-based assay system. Against the low passage clinical isolate HIV-1_{ROJO}, N-glycolylneuraminic acid was active with and without a 2hr pretreatment of the cells. Activity was strain-specific, however, as N-glycolylneuraminic acid was inactive against the laboratory strain HIV-1_{IIIB}. The TC₅₀ of N-glycolylneuraminic acid was greater than 1mM in the PBMC cell based assay using HIV-1_{ROJO or IIIB}, whereas for AZT, the TC₅₀ was greater than 4µM. The IC₅₀ of N-glycolylneuraminic acid was approximately 0.058mM without a pretreatment and 0.010mM with a 2hr pretreatment for HIV-1_{ROJO}, whereas the IC₅₀ for AZT was calculated to be 0.008µM without pretreatment and 0.004µM with a 2hr pretreatment. The IC₅₀ for AZT was 0.002µM for HIV-1_{IIIB}.

In this series of experiments, N-glycolylneuraminic acid did not have anti-viral activity against laboratory strains of HIV. It is possible that preservatives such as glycerol contained in these laboratory strains or diluents such as DMSO interfere with the anti-viral activity of N-glycolylneuraminic acid. See, Science, 1996, 274:1393-1395.

**Example 6-P-24 Assay for Infected PBMCs Treated with N-Glycolylneuraminic Acid:** PBMCs were isolated as described above and incubated with HIV-1 MN (NIH AIDS reagent program, catalog #137) at 37°C for 3 hours in 15mL tubes. Test groups included uninfected PBMCs, infected PBMCs with no N-glycolylneuraminic acid, and infected PBMCs plus N-glycolylneuraminic acid. Concentration of N-glycolylneuraminic acid ranged from about 6.2µg (1:2 dilution) to about 50µg (1:64 dilution). Excess virus was removed by washing the cells with PBS one time then the cells were exposed to N-glycolylneuraminic acid. P24 output was measured using the DuPont P24 assay kit (optical density (OD) in Table 15). Syncytia formation was examined as well as cytopathic effects (CPE). Mean p24 output was 9.68 x10⁶ ± 3.24 x10⁶ pg HIV-1P24/ml in unreacted samples (no N-glycolylneuraminic acid) and 624± 326 pg HIV-1P24/ml in samples treated with N-glycolylneuraminic acid (corrected for dilution factor). Sample #7 (p24 123x10⁶ pg HIV-1P24/ml) was excluded from the mean P24 output calculation due to a sampling error. In unreacted samples, cell viability was 19%, whereas in samples treated with N-glycolylneuraminic acid, cell viability was 25%. As indicated in Table 15, syncytia formation and HIV-induced cytopathic effects were prevented in infected cells by treatment with N-glycolylneuraminic acid.

**TABLE 15**

| **P24 output in Infected PBMCs** | | | | |
|---|---|---|---|---|
| **Sample #** | **O.D.** | **P-24*** | **Syncytia** | **CPE** |
| 1-4 Uninfected-PBMCs | 0.099 | 70 | negative | negative |
| | 0.117 | 89 | negative | negative |
| | 0.055 | 135 | negative | negative |
| | 0.012 | 90 | negative | negative |
| 5-10 Infected-PBMCs-no Neu5Gc | 0.469 | 14.7 x10⁶ | positive | 4+ |
| | 0.417 | 10.9 x10⁶ | positive | 4+ |
| | 0.235 | 6.66 x10⁶ | positive | 4+ |
| | 0.389 | 8.82 x10⁶ | positive | 4+ |
| | 0.249 | 7.3 x10⁶ | positive | 4+ |
| 11-16 Infected-PBMCs-Neu5Gc 1:2 | 0.017 | 320 | negative | negative |
| 1:4 | 0.043 | 922 | negative | negative |
| 1:8 | 0.057 | 871 | negative | negative |
| 1:16 | 0.061 | 824 | negative | negative |
| 1:32 | 0.012 | 265 | negative | negative |
| 1:64 | 0.041 | 865 | negative | negative |

| | | | | |
|---|---|---|---|---|
| *P-24 as picograms HIV-1 p24 per mL | | | | |

**Example 8-Comparison of Inhibition of LUKOR, N-Glycolylneuraminic Acid, and N-Acetylneuraminic Acid:** The RT assay described in Example 5 was used to compare the inhibitory activity of LUKOR, N-glycolylneuraminic acid, and N-acetylneuraminic acid. PBMCs were treated with dilutions of LUKOR of 1:4, 1:20, 1:100, 1:500, or 1:2500, or concentrations ofN-glycolylneuraminic acid or N-acetylneuraminic acid of 0.4, 1.2, 3.6, 11, or 33µg/ml. No carbohydrates were added to the control sample. RT activity was determined at 3, 6, 9,13, 17, and 20 days post-infection (PI) with X4 strain LAI/IIIB.

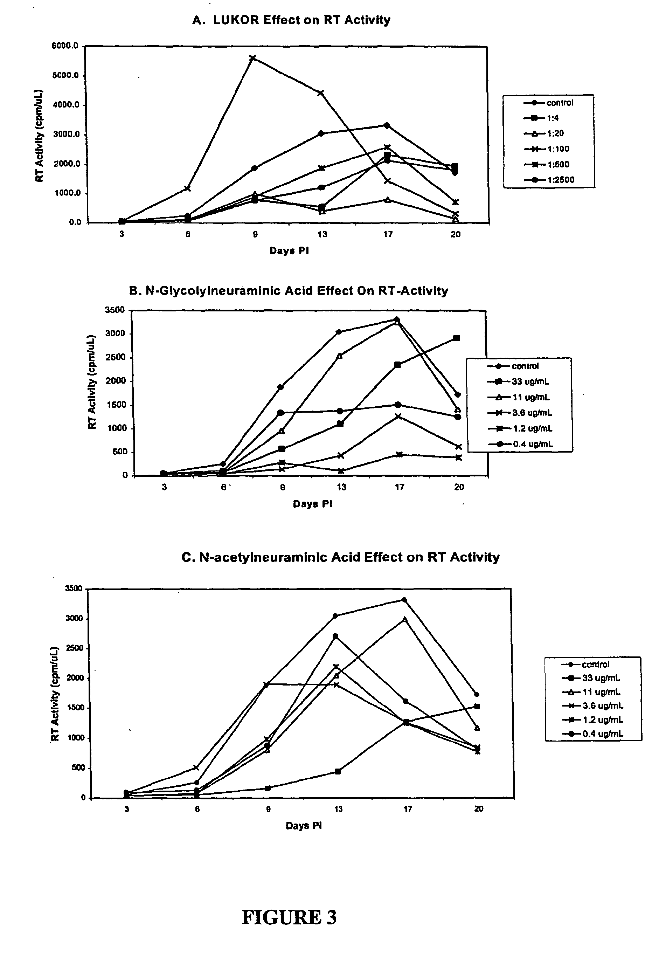

Results are shown in Table 17. RT activity was decreased in cells treated with LUKOR, with the 1:4 and 1:20 dilutions of LUKOR most effective. In N-glycolylneuraminic acid treated samples, RT activity was decreased, especially with the middle concentrations (1.2 and 3.6µg/ml). In contrast, decreased RT activity was observed only with high concentrations of N-acetylneuraminic acid. Figures 3A-3C are graphs that illustrate the inhibitory properties.

**TABLE 17**

| **RT Activity in Infected Cells Treated with Carbohydrates** | | | | | | |
|---|---|---|---|---|---|---|
| **Days PI** | **Control** | **LUKOR** | | | | |
| | | **1:4** | **1:20** | **1:100** | **1:500** | **1:2500** |
| 3 | 56 | 34 | 81 | 66 | 21 | 51 |
| 6 | 255 | 117 | 116 | 1180 | 81 | 73 |
| 9 | 1879 | 783 | 989 | 5615 | 891 | 765 |
| 13 | 3046 | 556 | 409 | 4421 | 1874 | 1211 |
| 17 | 3312 | 2326 | 795 | 1453 | 2580 | 2131 |
| 20 | 1723 | 1940 | 143 | 3250 | 719 | 1808 |

| **Days PI** | **Control** | **N-glycolylneuraminic acid µg/ml** | | | | |
|---|---|---|---|---|---|---|
| | | **33** | **11** | **3.6** | **1.2** | **0.4** |
| 3 | 56 | 32 | 40 | 45 | 35 | 53 |
| 6 | 255 | 75 | 75 | 44 | 39 | 113 |
| 9 | 1879 | 567 | 956 | 139 | 280 | 1342 |
| 13 | 3046 | 1103 | 2544 | 428 | 100 | 1371 |
| 17 | 3312 | 2356 | 3261 | 1262 | 449 | 1509 |
| 20 | 1723 | 2917 | 1411 | 616 | 385 | 1250 |

| **Days PI** | **Control** | **N-acetylneuraminic acid µg/ml** | | | | |
|---|---|---|---|---|---|---|
| | | **33** | **11** | **3.6** | **1.2** | **0.4** |
| 3 | 56 | 36 | 43 | 83 | 34 | 89 |
| 6 | 255 | 53 | 75 | 508 | 83 | 129 |
| 9 | 1879 | 165 | 799 | 1902 | 982 | 878 |
| 13 | 3046 | 442 | 2047 | 1892 | 2192 | 2703 |
| 17 | 3312 | 1273 | 2990 | 1260 | 1246 | 1615 |
| 20 | 1723 | 1526 | 1171 | 836 | 762 | 812 |

**Example 10 - Inhibition of gp120 binding:** The effect of N-glycolylneuraminic acid on preventing binding of HIV_{MN} to CEM-SS cells was assessed. Concentrations of N-glycolylneuraminic acid ranged from 10-200µm/ml. Saline was used as the control. Results are reported in Table 19 as percent inhibition of gp120 binding. Inhibition of binding was dose-dependent.

**TABLE 19**

| **Inhibition of gp120 binding** | |
|---|---|
| **Conc. of N-Glycolylneuraminic Acid** | **Inhibition of gp120 Binding** |
| **(µm/ml)** | **(% of control)** |
| 10 | 3.4 |
| 20 | 9.8 |
| 40 | 34.1 |
| 80 | 57.6 |
| 200 | 86.8 |

## Claims

1. Use of N-glycolylneuraminic acid, a sulphated N-glycolylneuraminic acid, a phosphorylated N-glycolylneuraminic acid, or a N-glycolylneuraminic salt, in the manufacture of a pharmaceutical composition for use in a treatment of an infection by a HIV virus of a human subject or prophylaxis of an infection by a HIV virus of a human subject.

2. Use according to claim 1, wherein said N-glycolymeuraminic acid, sulphated N-glycolylneuraminic acid, phosphorylated N-glycolylneuraminic acid, or N-glycolylneuraminic salt is to be administered by a route selected from intravenous administration, subcutaneous administration, oral administration, inhalation, or transdermal administration

3. Use according to claim 1, wherein said subject is monitored for the presence of the viral infection by determining viral load.

4. Use according to claim 1, wherein said N-glycolylneuraminic acid, sulphated N-glycolylneuraminic acid phosphorylated N-glycolylneuraminic acid, or N-glycolylneuraminic salt is to be administered in an amount between 1 mg and 1000 mg per administration.

5. Use according to claim 1, wherein said N-glycolylneuraminic acid, sulphated N-glycolylneuraminic acid, phosphorylated N-glycolylneuraminic acid, or N-glycolylneuraminic salt is to be administered in an amount between 10 mg and 100 mg per administration

6. Use according to claim 1, wherein said N-glycolylneuraminic acid, sulphated N-glycolylneuraminic acid, phosphorylated N-glycolylneuraminic acid, or N-glycolylneuraminic sah is to be administered in an amount between 30 mg and 80 mg per administration.

7. Use according to any of claims 4,5 or 6 wherein said N-glycolylneuraminic acid, sulphated N-glycolylneuraminic acid, phosphorylated N-glycolylneuraminic acid, or N-glycolylneuraminic salt is to be administered daily.

8. Use according to claim 1, wherein N-glycolylneuraminic acid is used and said N-glycolyneuraminic acid is synthetic or derived from a biological sample.

9. Use according to claim 1, wherein sulphated N-glycolylneuraminic acid or phosphorylated N-glycolylneuraminic acid is used.

10. Use according to claim 1, wherein a second anti-viral agent is to be administered.

11. Use according to claim 10, wherein said second anti-viral agent is conjugated to said N-glycolylneuraminic acid, sulphated N-glycolylneuraminic acid, phosphorylated N-glycolylneuraminic acid, or N-glycolylneuraminic salt.

12. Use according to claim 10, wherein said second anti-viral agent is a reverse transcriptase inhibitor or a protease inhibitor

## Patentansprüche

1. Verwendung von N-Glycolylneuraminsäure, einer sulfatierten N-Glycolylneuraminsäure, einer phosphorylierten N-Glycolylneuraminsäure oder einem N-Glycolylneuraminsäuresalz bei der Herstellung einer pharmazeutischen Zusammensetzung zur Verwendung bei einer Behandlung einer Infektion durch einen HIV-Virus bei einem menschlichen Patienten oder der Vorbeugung vor einer Infektion durch einen HIV-Virus bei einem menschlichen Patienten.

2. Verwendung nach Anspruch 1, bei der die N-Glycolylneuraminsäure, sulfatierte N-Glycolylneuraminsäure, phosphorylierte N-Glycolylneuraminsäure oder das N-Glycolylneuraminsäuresalz über einen Weg zu verabreichen ist, der aus intravenöser Verabreichung, subkutaner Verabreichung, oraler Verabreichung, Inhalation oder transdermaler Verabreichung ausgewählt wird.

3. Verwendung nach Anspruch 1, bei der die Person im Hinblick auf das Vorliegen der viralen Infektion durch Bestimmung der Viruslast kontrolliert wird.

4. Verwendung nach Anspruch 1, bei der die N-Glycolylneuraminsäure, sulfatierte N-Glycolylneuraminsäure, phosphorylierte N-Glycolylneuraminsäure oder das N-Glycolylneuraminsäuresalz in einer Menge zwischen 1 mg und 1.000 mg pro Verabreichung zu verabreichen ist.

5. Verwendung nach Anspruch 1, bei der die N-Glycolylneuraminsäure, sulfatierte N-Glycolylneuraminsäure, phosphorylierte N-Glycolylneuraminsäure oder das N-Glycolylneuraminsäuresalz in einer Menge zwischen 10 mg und 100 mg pro Verabreichung zu verabreichen ist.

6. Verwendung nach Anspruch 1, bei der die N-Glycolylneuraminsäure, sulfatierte N-Glycolylneuraminsäure, phosphorylierte N-Glycolylneuraminsäure oder das N-Glycolylneuraminsäuresalz in einer Menge zwischen 30 mg und 80 mg pro Verabreichung zu verabreichen ist.

7. Verwendung nach irgendeinem der Ansprüche 4, 5 oder 6, bei der die N-Glycolylneuraminsäure, sulfatierte N-Glycolylneuraminsäure, phosphorylierte N-Glycolylneuraminsäure oder das N-Glycolylneuraminsäuresalz täglich zu verabreichen ist.

8. Verwendung nach Anspruch 1, bei der N-Glycolylneuraminsäure verwendet wird und die N-Glycolylneuraminsäure synthetisch ist oder von einer biologischen Probe stammt.

9. Verwendung nach Anspruch 1, bei der sulfatierte N-Glycolylneuraminsäure oder phosphorylierte N-Glycolylneuraminsäure verwendet wird.

10. Verwendung nach Anspruch 1, bei der ein zweites antivirales Mittel zu verabreichen ist.

11. Verwendung nach Anspruch 10, bei der das zweite antivirale Mittel mit der N-Glycolylneuraminsäure, sulfatierten N-Glycolylneuraminsäure, phosphorylierten N-Glycolylneuraminsäure oder dem N-Glycolylneuraminsäuresalz konjugiert ist.

12. Verwendung nach Anspruch 10, bei der das zweite antivirale Mittel ein Reverse-Transkriptase-Hemmer oder ein Proteasehemmer ist.

## Revendications

1. Utilisation de l'acide N-glycolylneuraminique, d'un acide N-glycolylneuraminique sulfaté, d'un acide N-glycolylneuraminique phosphorylé, ou d'un sel N-glycolylneuraminique, dans la production d'une composition pharmaceutique destinée à être employée dans un traitement d'une infection par un virus de l'immunodéficience humaine (VIH) chez un sujet humain, ou la prophylaxie d'une infection par un virus VIH chez un sujet humain.

2. Utilisation selon la revendication 1, dans laquelle ledit acide N-glycolylneuraminique, ledit acide N-glycolylneuraminique sulfaté, ledit acide N-glycolylneuraminique phosphorylé ou ledit sel N-glycolylneuraminique, est destiné à être administré par une voie choisie parmi l'administration intraveineuse, l'administration sous cutanée, l'administration orale, l'inhalation ou l'administration transdermique.

3. Utilisation selon la revendication 1, dans laquelle ledit sujet est contrôlé pour la présence de l'infection virale par détermination de la charge virale.

4. Utilisation selon la revendication 1, dans laquelle ledit acide N-glycolylneuraminique, ledit acide N-glycolylneuraminique sulfaté, ledit acide N-glycolylneuraminique phosphorylé ou ledit sel N-glycolylneuraminique, est destiné à être administré selon une quantité entre 1 mg et 1000 mg par administration.

5. Utilisation selon la revendication 1, dans laquelle ledit acide N-glycolylneuraminique, ledit acide N-glycolylneuraminique sulfaté, ledit acide N-glycolylneuraminique phosphorylé ou ledit sel N-glycolylneuraminique, est destiné à être administré selon une quantité entre 10 mg et 100 mg par administration.

6. Utilisation selon la revendication 1, dans laquelle ledit acide N-glycolylneuraminique, ledit acide N-glycolylneuraminique sulfaté, ledit acide N-glycolylneuraminique phosphorylé ou ledit sel N-glycolylneuraminique, est destiné à être administré selon une quantité entre 30 mg et 80 mg par administration.

7. Utilisation selon l'une quelconque des revendications 4, 5 ou 6, dans laquelle ledit acide N-glycolylneuraminique, ledit acide N-glycolylneuraminique sulfaté, ledit acide N-glycolylneuraminique phosphorylé ou ledit sel N-glycolylneuraminique, est destiné à être administré quotidiennement.

8. Utilisation selon la revendication 1, dans laquelle ledit acide N-glycolylneuraminique est employé, et ledit acide N-glycolylneuraminique est synthétique ou dérivé d'un échantillon biologique.

9. Utilisation selon la revendication 1, dans laquelle l'acide N-glycolylneuraminique sulfaté ou l'acide N-glycolylneuraminique phosphorylé est employé.

10. Utilisation selon la revendication 1, dans laquelle un deuxième agent antiviral doit être administré.

11. Utilisation selon la revendication 10, dans laquelle ledit deuxième agent antiviral est conjugué avec ledit acide N-glycolylneuraminique, ledit acide N-glycolylneuraminique sulfaté, ledit acide N-glycolylneuraminique phosphorylé ou ledit sel N-glycolylneuraminique.

12. Utilisation selon la revendication 10, dans laquelle ledit deuxième agent antiviral est un inhibiteur de transcriptase inverse ou un inhibiteur de protéase.
